# EUROPÄISCHE PATENTSCHRIFT

(11) **EP 1 173 136 B1**
(45) Veröffentlichungstag und Bekanntmachung des Hinweises auf die Patenterteilung: **19.11.2003**
(21) Anmeldenummer: 99973828.9
(22) Anmeldetag: 29.10.1999
(51) Int. Cl.: A61K 6/04, C22C 19/07

(54) **DENTALAUFBRENNLEGIERUNG**
DENTAL BURNING-ON ALLOY
ALLIAGE DE CUISSON A USAGE DENTAIRE

(30) Priorität: 23.04.1999 DE 19918426
(43) Veröffentlichungstag der Anmeldung: 23.01.2002
(73) Patentinhaber: Ahlden, Manfred, 29664 Walsrode (DE); Petroll, Claudia, 21723 Hollern-Twielenfleth (DE)
(72) Erfinder: AHLDEN, Manfred, D-29664 Walsrode (DE); PETROLL, Claudia, D-21723 Hollern (DE)
(74) Vertreter: Hansen, Jochen
(86) Internationale Anmeldenummer: EP9908209
(87) Internationale Veröffentlichungsnummer: WO00064403

(56) Entgegenhaltungen:
- EP-A- 0 213 781
- WO-A-82/03007
- WO-A-85/04576
- DE-C- 3 415 249
- DE-C- 3 436 118

## Beschreibung

Die vorliegende Erfindung betrifft eine Dentalaufbrennlegierung für die Zahntechnik sowie die Verwendung dieser Dentalaufbrennlegierung für die Herstellung von insbesondere Dentalimplantaten, Brücken, Kronen, kombiniertem Zahnersatz etc.

In der Zahntechnik werden neben den teueren Edelmetalllegierungen Nicht-Edelmetalllegierungen (nachstehend NEM-Legierungen genannt) verwendet, die wesentlich billiger sind, jedoch eine Reihe von Nachteilen bei der Verarbeitung und Handhabung aufweisen.

Ein wesentlicher Bestandteil von NEM-Legierungen ist Chrom. Es hat sich jedoch gezeigt, dass bei den bei der Verarbeitung dieser Legierungen eingesetzten Brennvorgängen Chrom maßgeblich oxidiert wird und zu unerwünschten grünlichen Verfärbungen der Legierungen bzw. der daraus hergestellten Produkte führt.

Zudem weisen die bekannten NEM-Legierungen eine nicht immer befriedigende Haftfestigkeit mit denen bei der Herstellung von Dentalimplantaten verwendeten Keramikmaterialien auf. Es bestand daher ein Bedarf Legierungen auf NEM-Basis herzustellen, die einen verbesserten Metall-Keramik-Verbund gewährleisten können.

Herkömmliche NEM-Legierungen sind zudem nur schlecht verarbeitbar, da sie bei den bei der Verarbeitung notwendigen Schmelzvorgängen auf der Oberfläche der geschmolzenen Legierung eine Gießhaut aus Oxiden der Legierungsbestandteile ausbilden, die bei der weiteren Verarbeitung problematisch ist, da Abrisse dieser Gießhaut mit in die Gussformen gerissen werden und so zu Defekten und Produkten mit ungenügenden Eigenschaften führen können.

Aufgrund ihrer schlechten Korrosionseigenschaften, die zu einem Herauslösen von Ionen aus der Legierung führen, können die herkömmlichen Legierungen allergene Reaktionen auslösen.

Es war daher Aufgabe der vorliegenden Erfindung eine Dentalaufbrennlegierung auf Basis von NEM-Legierungen zur Verfügung zu stellen, die die genannten Nachteile nicht aufweist.

Erfindungsgemäß wird diese Aufgabe gelöst durch eine Dentalzusammensetzung nach Anspruch 1.
Die Unteransprüche betreffen besondere Ausgestaltungen der Dentalaufbrennlegierung nach dem Anspruch 1.

Für die vorliegende Erfindung beziehen sich die Prozentangaben auf den Gewichtsanteil der jeweiligen Komponente bezogen auf die Gesamtlegierung.

So betrifft die Erfindung eine Dentalaufbrennlegierung im Wesentlichen enthaltend Kohlenstoff 0,0 bis 0,4 %, Mangan 0,1 bis 1,0 %, Silizium 0,1 bis 1,0%, Chrom 20,0 bis 30,0 %, Nickel 0,0 bis 1,0 %, Molybdän 3,0 bis 7,0 %, Eisen 0,0 bis 0,75 %, Gold 0,1 bis 5,0 %, Platin 0,1 bis 4,0%, Rest Kobalt sowie gegebenenfalls unvermeidliche Verunreinigungen.

Vorzugsweise ist der Anteil an Chrom 24,0 % bis 30,0 % und der an Molybdän 4,0 bis 7,0 %.

Es versteht sich, dass die erfindungsgemäße Dentalaufbrennlegierung neben den in den Ansprüchen genannten Bestandteilen auch Spuren von unvermeidlichen Verunreinigungen enthalten kann, wie sie zum Beispiel in den Ausgangsmaterialien vorhanden sein können oder die im Zuge der Verarbeitung eingeschleppt werden.

Vorzugsweise ist in der Legierung der Anteil Mangan geringer als der Anteil Silizium.

Wesentlich ist, dass die Legierung Gold in einem Anteil von 0,1 bis 5,0 % und Platin in einem Anteil von 0,1 bis 4,0 % enthält. Vorzugsweise beträgt der Goldanteil maximal 4,0 %, insbesondere maximal 2,5 %, und der Platinanteil maximal 2,5 %.

Gemäß einer weiteren Ausführungsform kann die erfindungsgemäße Legierung Gallium und/oder Indium in einem Anteil von 0,1 % bis 16 % enthalten.

Besonders bevorzugt enthält die erfindungsgemäße Dentalaufbrennlegierung Gallium und/oder Indium in einem Anteil von je 0,1 bis 8,5 %, vorzugsweise bis 5,0 % und besonders bevorzugt bis 3,5 %. In einer weiteren Ausführungsform enthält die erfindungsgemäße Dentalaufbrennlegierung Gallium in einem Anteil von 0,1 bis 3,5 % und/oder indium in einem Anteil von 0,1 bis 3,5 %. Eine besonders bevorzugte Untergrenze für den vorstehend genannten Gesamtbereich für Gallium und/oder Indium ist 0,2 %.

Vorteilhafte Legierungen lassen sich auch erhalten, wenn der Gallium und/oder Indiumanteil in einem Bereich von jeweils 3,5 % bis 6,5 % eingestellt wird.

Des Weiteren kann die Erfindung Tantal und/oder Niob mit einem Anteil von 0,1 bis 2,0 %, vorzugsweise von 0,1 bis 1,0 % Tantal und/oder 0,1 bis 1,0 % Niob enthalten.

Gemäß einer weiteren bevorzugten Ausführungsform wird der Erfindung mindestens ein Seltenerdelement zugesetzt. Bevorzugt sind Cer, Yttrium und Zirkon in einem Anteil von 0,1 bis 3,0 %. Bevorzugte Bereiche sind für Cer 0,1 bis 1,0 % und/oder für Yttrium 0,1 bis 1,0 % und/oder für Zirkon 0,1 bis 1,0 %.

Die Zugabe von Gold und Platin vermindert eine übermäßige Oxidation von Chrom während der Brennvorgänge (Chromwachstum) wesentlich, so dass unerwünschte Verfärbungen der Produkte nicht mehr auftreten. Des Weiteren bewirkt die Verminderung des Oxidwachstums eine Erhöhung der Haftfestigkeit der Legierung in einem Metall-Keramik-Verbund.

Aufgrund der Edelmetalizugabe verbessert sich zudem die Lötbarkeit der erfindungsgemäßen Legierung, die ohne weiteres mit handelsüblichen Loten zusammen verwendet werden kann und metallurgisch einwandfreie Metallverbindungen ermöglicht.

Durch den Zusatz von Gallium und/oder Indium läßt sich der Schmelzpunkt der Legierung erniedrigen, so dass insbesondere ein Schmelzintervall von 1320 bis 1340 °C erzielt werden kann. Durch Einstellung des Schmelzintervalls auf diesen niedrigen Bereich läßt sich die erfindungsgemäße Legierung auch ohne weiteres in handelsüblichen widerstandsbeheizten Gießöfen für die Zahntechnik und daneben auch in Hochfrequenzgießmaschinen verarbeiten.

Die Erniedrigung des Schmelzpunktes bewirkt zudem, dass bei der Verarbeitung mit insbesondere Siliziumhaltigen Materialien kein Durchschlagen von Si-Körnern erfolgt, die die Qualität der Legierung verringern könnten.

Es hat sich zudem gezeigt, dass die erfindungsgemäße Legierung besonders dünnflüssig ist und auch für die Verarbeitung zu filigranen Konfigurationen wie Hütchen in extrem dünnen Wandstärken geeignet ist. So konnten mit der erfindungsgemäßen Legierung Wandstärken von nur 0,15 mm erzielt werden. Trotz dieser dünnen Wandstärken zeichnet sich die erfindungsgemäße Legierung durch gute Festigkeit und Stabilität aus. Durch die dünne Ausgestaltung von Kronen und Hütchen kann insbesondere Zahnfleischreizung, wie sie bei dicken Kronenrändern auftreten kann, im zervikalen Bereich verhindert werden.

Die Zugabe von mindestens einem Seltenerdelement bewirkt eine zusätzliche Erhöhung der Haftfestigkeit der erfindungsgemäßen Legierung im Metall-Keramik-Verbund.
Insbesondere durch den kombinierten Zusatz von Gold und Platin sowie mindestens einem Seltenerdelement lassen sich für einen Metall-Keramik-Verbund Festigkeiten erreichen, die selbst bei starken Hammerstößen nach dem Hammerstoßtest nach Homann (DIN 13912) den Verbund nicht zum Brechen bringen.

Den erfindungsgemäßen Legierungen kann zudem Zinn in einem Anteil von bis zu 1,5 % zugesetzt werden, wodurch eine Verbesserung der Haftung an das Keramikmaterial erzielt werden kann.

Die erfindungsgemäßen Legierungen können ohne weiteres mit herkömmlichen handelsüblichen Bondermaterialien (Haftgrundvermittlern) verarbeitet werden.
Sie eignen sich zur Herstellung von beliebigen Dentalimplantaten, Kronen, Brücken, kombiniertem Zahnersatz etc. Sie können auch hervorragend für die Herstellung von Dentalimplantaten mit entsprechenden Beschichtungen wie z.B. Tricalciumphosphatat, Hydroxylapatit und anderen biokompatiblen knochenfreundlichen Schichten gegebenenfalls zusammen mit Bondern verwendet werden.

Ein besonderer Vorteil der erfindungsgemäßen Legierung ist ihre leichte Handhabbarkeit und Verarbeitbarkeit. Wie bereits ausgeführt bildet sie keine Gießhaut aus und aufgrund der Oxidreduzierung wird ein Ankleben der Schmelze an die Dentalgussform verhindert. Des Weiteren zeichnet sie sich durch eine extrem leichtgießende Schmelze aus.
Es hat sich auch gezeigt, dass die sonst üblichen Volumenkontraktionen beim Erstarren nach dem Guss verringert werden und somit extrem passgenaue Gusse erhalten werden können.
Auch die mechanischen Eigenschaften konnten gegenüber den bekannten Legierungen deutlich verbessert werden, wobei in Abhängigkeit von der Gussführung Werte von bis zu 18 % Bruchdehnung erreicht werden konnten.

Für die erfindungsgemäßen Legierungen sind zudem keine allergenen Reaktionen bekannt.

Ein generelles Problem bei der Herstellung von Metall-Keramik-Verbunden ist das in der Regel unterschiedliche Wärmeausdehnungsverhalt der Metalllegierung einerseits und des Keramikmaterials andererseits, aufgrund dessen infolge schlecht übereinstimmender Materialkombinationen häufig einhergehend mit ungeeigneten Brenn- und Abkühlmethoden Sprünge oder Abplatzungen auftreten können. Gewünscht ist jedoch ein Verbund zwischen Metall und Keramik von möglichst unbegrenzter Dauer, der nicht durch Sprünge oder Abplatzungen in der Keramik zerstört wird.

Es hat sich gezeigt, dass sich der Wärmeausdehnungskoeffizient der erfindungsgemäßen Legierungen ohne weiteres auf einen Wert einstellen lässt, der dem Wärmeausdehnungskoeffizienten der üblicherweise verwendeten Keramiken entspricht, der in der Größenordnung von 15,9 x 10⁻⁶ (bei 500 °C; gemäß DIN EN ISO 9693) liegt.

Gemäß einer besonders bevorzugten Ausführungsform weist daher die erfindungsgemäße Legierung einen Wärmeausdehnungskoeffizienten auf, der dem Wärmeausdehnungskoeffizienten des verwendeten Keramikmaterials gleich oder ähnlich ist. Vorzugsweise hat die erfindungsgemäße Legierung einen Wärmeausdehnungskoeffizienten von 15,7 x 10⁻⁶ bis 16,2 x 10⁻⁶, insbesondere in etwa 15,9 x 10⁻⁶ (bei 500 °C).

Aufgrund ihrer vorteilhaften Eigenschaften eignet sich die erfindungsgemäße Legierung auch für niedrig schmelzende Keramikmassen wie sie heutzutage in erblichen Ausmaß verwendet werden.

Die Herstellung und Verarbeitung der Legierung erfolgt nach den üblichen dafür bekannten Verfahren und Methoden.

Bei der Verarbeitung lassen sich durch Verwendung von hochreinen Ausgangsstoffen und durch Einstellen eines extrem feinkristallinen Gefüges hoch zäheelastische Legierungen erhalten, die ohne Probleme drucklos bearbeitet werden können und zudem außerordentlich gut polierbar sind

Aufgrund des mit der erfindungsgemäßen Legierungen erzielbaren Gießpunktes über dem Solidus-Liquiduspunkt lässt sich ein mögliches Durchbiegen bei der Erhitzung vermeiden, wie es häufig bei üblichen Legierungen auftritt. Wie bereits vorstehend erwähnt, kann so die erfindungsgemäße Legierung in den im Dentalgewerbe üblichen Gießöfen ohne weiteres erschmolzen werden, wobei bevorzugt die Gießtemperaturen in einem Bereich von 1420 °C liegen.

Darüber hinaus hat es sich als vorteilhaft erwiesen, Legierungen mit geringer Dichte auszuwählen, da dadurch ebenfalls einem Verziehen des Metalls entgegengewirkt werden kann.

## Patentansprüche

1. Dentalaufbrennlegierung
im Wesentlichen enthaltend
Kohlenstoff 0,0 bis 0,4 %, Mangan 0,1 bis 1,0 %, Silizium 0,1 bis 1,0 %, Chrom 20,0 bis 30,0 %, Nickel 0,0 bis 1,0 %, Molybdän 3,0 bis 7,0 %, Eisen 0,0 bis 0,75 %, Gold 0,1 bis 5,0 %, Platin 0,1 bis 4,0 %, Rest Kobalt sowie gegebenenfalls unvermeidliche Verunreinigungen.

2. Dentalaufbrennlegierung nach Anspruch 1,
wobei der Anteil an Chrom 24,0 bis 30,0 % und/oder der an Molybdän 4,0 % bis 7,0 % beträgt.

3. Dentalaufbrennlegierung nach Anspruch 1 oder 2,
wobei der Anteil an Gold 0,1 bis 4,0 % beträgt.

4. Dentalaufbrennlegierung nach Anspruch 2,
wobei der Anteil an Gold maximal 2,5 % beträgt.

5. Dentalaufbrennlegierung nach einem der vorhergehenden Ansprüche,
wobei der Anteil an Platin 0,1 bis 2,5 % beträgt.

6. Dentalaufbrennlegierung nach einem der vorhergehenden Ansprüche,
zusätzlich enthaltend
Gallium und/oder Indium in einem Anteil von 0,1 bis 16,0 %.

7. Dentalaufbrennlegierung nach Anspruch 6,
wobei der Anteil an Gallium und/oder Indium 0,1 bis 10,0 % beträgt.

8. Dentalaufbrennlegierung nach Anspruch 6,
wobei der Anteil an Gallium und/oder Indium 0,1 bis 7,0 % beträgt.

9. Dentalaufbrennlegierung nach einem der Ansprüche 6 oder 7,
wobei Gallium in einem Anteil von 0,1 bis 8,5 % und/oder Indium in einem Anteil von 0,1 bis 8,5 % enthalten sind.

10. Dentalaufbrennlegierung nach einem der vorhergehenden Ansprüche,
zusätzlich enthaltend Tantal und/oder Niob in einem Anteil von 0,1 bis 2,0 %.

11. Dentalaufbrennlegierung nach Anspruch 10,
mit einem Anteil von Tantal von 0,1 bis 1,0 % und/oder einem Anteil an Niob von 0,1 bis 1,0 %.

12. Dentalaufbrennlegierung nach einem der vorhergehenden Ansprüche,
zusätzlich mindestens ein Element enthaltend, ausgewählt aus der Gruppe unter Cer, Yttrium und Zirkon mit einem Gesamtanteil von 0,1 bis 3,0 %.

13. Dentalaufbrennlegierung nach Anspruch 12,
enthaltend Cer mit einem Anteil von 0,1 bis 1,0 % und/oder Yttrium mit einem Anteil von 0,1 bis 1,0 % und/oder Zirkon mit einem Anteil von 0,1 bis 1,0 %.

14. Dentalaufbrennlegierung nach Anspruch 1 oder 2,
zusätzlich enthaltend Gallium 0,1 bis 8,0 %, Indium 0,1 bis 8,0 %, Tantal 0,1 bis 1,0 %, Cer 0,1 bis 1,0 %, Yttrium 0,1 bis 1,0 % und Zirkon 0,1 bis 1,0 %.

15. Dentalaufbrennlegierung nach Anspruch 14,
mit einem Anteil an Gold von 0,1 bis 4,0 % und/oder einem Anteil an Platin von 0,1 bis 2,5 %.

16. Dentalaufbrenniegierung nach Anspruch 14 oder 15,
mit einem Anteil an Gallium von 0,1 bis 5,0 % und/oder einem Anteil an Indium von 0,1 bis 5,0 %.

17. Dentalaufbrennlegierung nach Anspruch 16,
mit einem Anteil an Gallium von 0,1 bis 3,5 % und einem Anteil an Indium von 0,1 bis 3,5 %.

18. Dentalaufbrennlegierung nach einem der vorherigen Ansprüche
zusätzlich enthaltend Zinn in einem Anteil von bis zu maximal 1,5 %.

19. Dentalaufbrennlegierung nach einem der vorhergehenden Ansprüche,
wobei die Legierung einen Wärmeausdehnungskoeffizienten von 15,7 x 10⁻⁶ bis 16,2 x 10⁻⁶ (bei 500 °C) hat.

20. Nicht-therapeutische Verwendung einer Dentalaufbrennlegierung nach einem der vorhergehenden Ansprüche für die Zahntechnik.

21. Verwendung einer Dentalaufbrennlegierung nach einem der Ansprüche 1 bis 19 zur Herstellung von Dentalimplantaten, Kronen, Brücken und kombiniertem Zahnersatz.

## Claims

1. A burn-on dental alloy essentially containing
0.0 to 0.4% carbon, 0.1 to 1.0% manganese, 0.1 to 1.0% silicon, 20.0 to 30.0% chromium, 0.0 to 1.0% nickel, 3.0 to 7.0% molybdenum, 0.0 to 0.75% iron, 0.1 to 5.0% gold, 0.1 to 4.0% platinum, residual cobalt as well as possibly unavoidable impurities.

2. A burn-on dental alloy according to Claim 1,
wherein the proportion of chromium amounts to 24.0 to 30.0% and/or that of molybdenum amounts to 4.0% to 7.0%.

3. A burn-on dental alloy according to Claim 1 or 2,
wherein the proportion of gold amounts to 0.1 to 4.0%.

4. Burn-on dental alloy according to Claim 2,
wherein the proportion of gold amounts to a maximum of 2.5%.

5. Burn-on dental alloy according to any preceding claim,
wherein the proportion of platinum amounts to 0.1 to 2.5%.

6. A burn-on dental alloy according to any preceding claim, containing gallium and/or indium in a proportion of 0.1 to 16%.

7. A burn-on dental alloy according to Claim 6, wherein the proportion of gallium and/or indium amounts to 0.1 to 10.0%.

8. A burn-on dental alloy according to Claim 6,
wherein the proportion of gallium and/or indium amounts to 0.1 to 7.0%.

9. A burn-on dental alloy according to Claim 6 or 7,
wherein gallium is contained in a proportion of 0.1 to 8.5% and/or indium is contained in a proportion of 0.1 to 8.5%.

10. A burn-on dental alloy according to any preceding claim, additionally containing tantalum and/or niobium in a proportion of 0.1 to 2.0%.

11. A burn-on dental alloy according to Claim 10,
with a proportion of tantalum of 0.1 to 1.0% and/or a proportion of niobium of 0.1 to 1.0%.

12. A burn-on dental alloy according to any preceding claim, additionally containing at least one element selected from the group including cerium, yttrium and zirconium with a total proportion of 0.1 to 3.0%.

13. A burn-on dental alloy according to Claim 12, containing cerium with a proportion of 0.1 to 1% and/or yttrium with a proportion of 0.1 to 1% and/or zirconium with a proportion of 0.1 to 1%.

14. A burn-on dental alloy according to Claim 1 or 2, additionally containing 0.1 to 8.0% gallium, 0.1 to 8.0% indium, 0.1 to 1.0% tantalum, 0.1 to 1.0% cerium, 0.1 to 1.0% yttrium and 0.1 to 1.0% zirconium.

15. A burn-on dental alloy according to Claim 14,
with a proportion of gold of 0.1 to 4.0% and/or a proportion of platinum of 0.1 to 2.5%.

16. A burn-on dental alloy according to Claim 14 or 15,
with a proportion of gallium of 0.1 to 5.0% and/or a proportion of indium of 0.1 to 5.0%.

17. A burn-on dental alloy according to Claim 16,
with a proportion of gallium of 0.1 to 3.5% and a proportion of indium of 0.1 to 3.5%.

18. A burn-on dental alloy according to any preceding Claim, additionally containing tin in a proportion of up to a maximum of 1.5%.

19. A burn-on dental alloy according to any preceding claim,
wherein the alloy has a coefficient of thermal expansion of 15.7 x 10⁻⁶ to 16.2 x 10⁻⁶ (at 500°C).

20. Non-therapeutic use of a burn-on dental alloy according to any preceding Claim for dentistry.

21. Use of a burn-on dental alloy according to any of Claims 1 to 19 for the production of dental implants, crowns, bridges and combined dental prostheses.

## Revendications

1. Alliage dentaire contenant en substance carbone 0,0 à 0,4%, manganèse 0,1 à 1,0%, silicium 0,1 à 1,0%, chrome 20,0 à 30,0%, nickel 0,0 à 1,0%, molybdène 3,0 à 7,0%, fer 0,0 à 0,75%, or 0,1 à 5,0%, platine 0,1 à 4,0%, cobalt et le cas échéant impuretés inévitables.

2. Alliage dentaire selon la revendication 1 dans lequel la proportion de chrome est comprise entre 24,0 et 30,0% et/ou la proportion de molybdène est comprise entre 4,0 et 7,0%.

3. Alliage dentaire selon les revendications 1 et 2 dans lequel la proportion d'or est comprise entre 0,1 et 4,0%.

4. Alliage dentaire selon la revendication 2 dans lequel la proportion d'or atteint au maximum 2,5%.

5. Alliage dentaire selon l'une des revendications précédentes, la proportion de platine étant comprise entre 0,1 et 2,5%.

6. Alliage dentaire selon l'une des revendications précédentes, contenant en outre du gallium et/ou de l'indium en une proportion comprise entre 0,1 et 16,0%.

7. Alliage dentaire selon la revendication 6 dans lequel la proportion de gallium et/ou d'indium est comprise entre 0,1 et 10,0%.

8. Alliage dentaire selon la revendication 6 dans lequel la proportion de gallium et/ou d'indium est comprise entre 0,1 et 7,0%.

9. Alliage dentaire selon l'une des revendications 6 ou 7 dans lequel le gallium est compris en une proportion de 0,1 à 8,5% et/ou l'indium en une proportion de 0,1 à 8,5%.

10. Alliage dentaire selon l'une des revendications précédentes contenant en outre du tantale et/ou du niobium en une proportion comprise entre 0,1 et 2,0%.

11. Alliage dentaire selon la revendication 10 avec une proportion de tantale comprise entre 0,1 et 1,0% et/ou une proportion de niobium comprise entre 0,1 et 1,0%.

12. Alliage dentaire selon l'une des revendications précédentes, contenant en outre au moins un élément choisi dans le groupe contenant le Cer, l'yttrium et le zirconium en une proportion totale comprise entre 0,1 et 3,0%.

13. Alliage selon la revendication 12 contenant du Cer en une proportion comprise entre 0,1 et 1,0% et/ou de l'yttrium en une proportion comprise entre 0,1 et 1,0% et/ou du zirconium en une proportion comprise entre 0,1 et 1,0%.

14. Alliage dentaire selon la revendication 1 ou 2 contenant en outre : gallium 0,1 à 8,0%, indium 0,1 à 8,0%, tantale 0,1 à 1,0%, Cer 0,1 à 1,0%, yttrium 0,1 à 1,0% et zirconium 0,1 à 1,0%.

15. Alliage dentaire selon la revendication 14 avec une proportion d'or comprise entre 0,1 et 4,0% et/ou une proportion de platine comprise entre 0,1 et 2,5%.

16. Alliage dentaire selon la revendication 14 ou 15 avec une proportion de gallium comprise entre 0,1 et 5,0% et/ou une proportion d'indium comprise entre 0,1 et 5,0%.

17. Alliage dentaire selon la revendication 16 avec une proportion de gallium comprise entre 0,1 et 3,5% et une proportion d'indium comprise entre 0,1 et 3,5%.

18. Alliage dentaire selon l'une des revendications précédentes contenant en outre de l'étain en une proportion atteignant au maximum 1,5%.

19. Alliage dentaire selon l'une des revendications précédentes, l'alliage ayant un coefficient d'expansion thermique compris entre 15,7 x 10⁻⁶ et 16,2 x 10⁻⁶ (à 500°C).

20. Utilisation non-thérapeutique d'un alliage dentaire selon l'une des revendications précédentes pour la technique dentaire.

21. Utilisation d'un alliage dentaire selon l'une des revendications 1 à 19 pour la fabrication d'implants dentaires, de couronnes, de bridges et de substituts dentaires combinés.
